# EUROPEAN PATENT APPLICATION

(11) **EP 4 442 252 A1**
(43) Date of publication of application: **09.10.2024**
(21) Application number: 24167899.4
(22) Date of filing: 29.03.2024
(51) Int. Cl.: A61K 9/14, A61K 9/20, A61K 9/28, A61K 31/00

(54) **A TABLET FORMULATION OF A SOLID DISPERSION COMPRISING EMPAGLIFLOZIN**

(30) Priority: 04.04.2023 TR 202303669
(71) Applicant: SANOVEL ILAC SANAYI VE TICARET A.S., Istanbul 34460 (TR)
(72) Inventor: SUNEL, Fatih, Istanbul (TR); PEHLIVAN AKALIN, Nur, Istanbul (TR); ARMUT, Merve, Istanbul (TR)
(74) Representative: Genç Ilhan, Oznur

(57) **Abstract**

The present invention relates to a tablet composition comprising a tablet formulation comprising a solid dispersion of empagliflozin or a pharmaceutically acceptable salt thereof or crystalline polymorph thereof, wherein the solid dispersion further comprising at least one binder, at least one surfactant and solvent. The present invention also relates to a simple, rapid, cost effective, timesaving and industrially convenient method of preparing the formulation.

## Description

### Field of the Invention

The present invention relates to a tablet composition comprising a tablet formulation comprising a solid dispersion of empagliflozin or a pharmaceutically acceptable salt thereof or crystalline polymorph thereof, wherein the solid dispersion further comprising at least one binder, at least one surfactant and solvent. The present invention also relates to a simple, rapid, cost effective, time-saving and industrially convenient method of preparing the formulation.

### Background of the Invention

Empagliflozin is a potent inhibitor of sodium-glucose co-transporter type 2 (SGLT-2) and is therefore used to treat type 2 diabetes. It is currently approved for the treatment of type 2 diabetes and improvement of blood sugar control.

Empagliflozin is a white to yellowish non-hygroscopic crystalline solid, very slightly soluble in water (pH 1-7.4), slightly soluble in acetonitrile and ethanol, sparingly soluble in methanol, and practically insoluble in toluene. The chemical name of empagliflozin (1S)-1,5-anhydro-1-(4-chloro-3-{4-[(3S)-tetrahydrofuran-3-yloxy]benzyl}phenyl)-D- glucitol and its chemical structure is shown in the Formula- I.

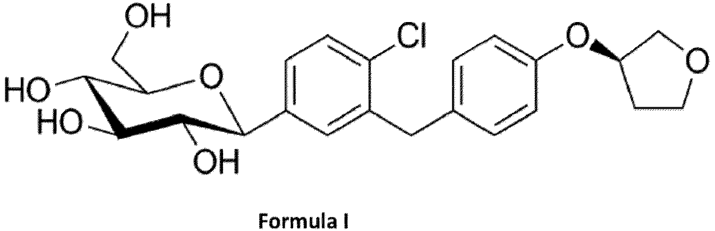

Empagliflozin is available on the market in the form of a free base and is sold under trade name Jardiance<^{®}>, as an oral tablet in 10 mg and 25 mg strengths. Further it is available on the market as a combination product with Metformin and a combination product with Linagliptin.

The first mention of the crystalline form of empagliflozin can be found in patent application WO 2006/117359. WO2006117360 discloses two other forms of empagliflozin which is described as Form I and Form II. Also, it disclosed methods for production thereof and synthesis of Empagliflozin.

US 20110014284 describes pharmaceutical compositions comprising empagliflozin, process for the preparation of such compositions and their use in the treatment of various conditions including type 1 diabetes mellitus and type 2 diabetes mellitus.

EP1730131 discloses Empagliflozin, process for production thereof, its use and pharmaceutical composition thereof. The composition of a tablet is disclosed that comprises active ingredient in admixture with pharmaceutically acceptable excipients, such as lactose, com starch, polyvinylpyrrolidone, magnesium stearate.

As evident from the prior art and the literature there are reported several pharmaceutical compositions comprising Empagliflozin. There is an existing and continual need for oral dosage formulations comprising Empagliflozin or a pharmaceutically acceptable salt thereof that have the desired dissolution profile, high stability, content uniformity, improved compressibility, flowability and manufactured by safe, effective, easy manufacturing methods.

Several pharmaceutical dosage forms of empagliflozin have been published. The main problem encountered when preparing formulations of empagliflozin is low solubility of empagliflozin, leading to difficulties with disintegration and dissolution times.

We found that the use of a solid dispersion carrier to empagliflozin in the tablet formulation provided the desired dissolution profile, homogeneity and flowability and even stability.

### Detailed Description of the Invention

The main object of the present invention is to provide a tablet formulation comprising Empagliflozin or a pharmaceutically acceptable salt thereof or crystalline polymorph thereof with the desired high dissolution profile.

Another object of the present invention is to provide a tablet formulation comprising Empagliflozin or a pharmaceutically acceptable salt thereof or crystalline polymorph thereof with improved stability, flowability and homogeneity.

Another object of the invention is a process of preparing a tablet formulation comprising empagliflozin or a pharmaceutically acceptable salt thereof or crystalline polymorph thereof. The process is wet granulation, which is a simple, rapid, cost effective, time-saving, and industrially convenient method.

Empagliflozin is poorly soluble in water. This causes problems of dissolution profile and bioavailability. In the present invention, some studies have been done so that these problems do not appear. Thanks to a solid dispersion at the present invention, the tablet which has a low solubility of empagliflozin or a pharmaceutically acceptable salt thereof or crystalline polymorph thereof has been developed with excellent pharmacochemical properties. Also, this provides the desired dissolution profile of the tablet. A homogeneous dispersion of empagliflozin has been provided thank to using the solid dispersion at least one binder and at least one surfactant with solvent in the solid dispersion, thus increasing the solubility and bioavailability of a tablet comprising empagliflozin. Also, in this formulation, we seen that even if used a surfactant in small quantities in the solid dispersion, this provides both stability and content uniformity.

According to one embodiment of this invention, a tablet formulation comprising a solid dispersion of empagliflozin or a pharmaceutically acceptable salt thereof or crystalline polymorph thereof wherein the solid dispersion further comprising at least one binder, at least one surfactant and solvent.

Suitable binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

According to this embodiment of the present invention, the amount of binders is between 1.0% and 10.0% by weight of the total core tablet. Preferably, the amount of binders is between 1.0% and 4.0% by weight of the total core tablet.

According to this embodiment of the present invention, the binder is hydroxypropyl cellulose. Preferably, it is hydroxypropyl cellulose LF.

Suitable surfactants are selected from the group comprising polysorbates (20,40,60,80,120), sodium lauryl sulfate, docusate sodium, glyceryl esters, glyceryl monoleate, poloxamer, polyethylene alkyl ethers, polyglyceryl esters, polyoxyetylene esters, polyoxyetylene stearates, sodium stearate, calcium oleate or mixtures thereof.

According to one embodiment of this invention, the surfactant is sodium lauryl sulfate or polysorbate. In this invention, this surfactant effectively improves the solubility of empagliflozin in a solvent and enable it to be quickly dispersed and dissolved.

According to one embodiment of this invention, the amount of surfactant is between 1.0% and 9.0% or 0.5% and 3.0% by weight in the total core tablet.

According to one embodiment of the present invention, the tablet formulation comprises a solid dispersion comprising;
- Empagliflozin or a pharmaceutically acceptable salt thereof or crystalline polymorph thereof
- HPC as a binder
- sodium lauryl sulfate or polysorbate as a surfactant.

According to one embodiment of the present invention, the amount of the solid dispersion is present between 5.0% and %25.0 by weight in the total core tablet.

According to one embodiment of the present invention, the amount of empagliflozin or a pharmaceutically acceptable salt thereof or crystalline polymorph thereof is present between 0.5% and %25.0 by weight in the total core tablet. Preferably, the amount of Empagliflozin is between 1.0% and 20.0% or 3.0% and 14.0% by weight in the total core tablet.

According to one embodiment of the present invention, preferably, empagliflozin is in the form of crystalline form.

Suitable solvents are selected from the group comprising pure water, isopropyl alcohol, propylene glycol, polyethylene glycol, glycerin, ethanol, or mixtures thereof.

According to one embodiment of the present invention, preferably the solvent is pure water or isopropyl alcohol or mixtures thereof.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agent and the excipients.

According to this embodiment of the present invention, the tablet composition further comprises at least one pharmaceutically acceptable excipient is selected from the group comprising disintegrants, fillers, lubricants, glidant or mixtures thereof.

Suitable disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

According to this embodiment of the present invention, the amount of disintegrants is between 1.0% and 10.0% by weight of the total core tablet. Preferably, the amount of disintegrants is between 1.0% and 4.0% by weight of the total core tablet.

According to this embodiment of the invention, the disintegrant is croscarmellose sodium.

Suitable fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals or mixtures thereof.

According to an embodiment of the present invention, the amount of fillers are 70.0% to 92.0% by weight in the total core tablet. Preferably, it is between 77.0% to 90.0% by weight in the total core tablet.

According to one embodiment of the present invention, the filler is microcrystalline cellulose. The amount of microcrystalline cellulose is 18.0% to 40.0% by weight in the total core tablet.

According to one embodiment of the present invention, the filler is lactose monohydrate. The amount of lactose monohydrate is 45.0% to 70.0% by weight in the total core tablet.

According to one embodiment of the present invention, the fillers are microcrystalline cellulose and lactose monohydrate.

Suitable lubricants are selected from the group comprising magnesium stearate, calcium stearate, zinc stearate, talc, boric acid, hydrogenated vegetable oil, sodium chlorate, magnesium lauryl sulfate, polyethylene glycol, stearic acid, fumaric acid or mixtures thereof.

According to this embodiment of the invention, the lubricant is magnesium stearate.

Suitable glidants are selected from the group comprising anhydrous colloidal silicon dioxide, calcium stearate, sodium chlorate, magnesium lauryl sulfate, sodium oleate, sodium acetate, sodium benzoate, polyethylene glycol, sodium lauryl sulfate, aluminum silicate, colloidal silica, calcium silicate or mixtures thereof.

According to this embodiment of the invention, the glidant is anhydrous colloidal silicon dioxide.

According to an embodiment of the present invention, the tablet composition comprises;
a) Empagliflozin or a pharmaceutically acceptable salt thereof or crystalline polymorph thereof
b) Microcrystalline Cellulose
c) Lactose monohydrate
d) HPC
e) Croscarmellose sodium
f) A surfactant (SLS or Tween)
g) Anhydrous colloidal silicon dioxide
h) Magnesium stearate

According to one embodiment of the present invention, the tablet formulation comprising a solid dispersion of empagliflozin or crystalline polymorph thereof is obtained by wet granulation.

According to one embodiment of the present invention, a process for the preparation of the tablet formulation comprises;
Obtained solid dispersion
   a) Mixing surfactant (SLS or polysorbate), HPC, empagliflozin and a solvent (pure water or isopropyl alcohol) and obtained solid dispersion,
Preparing a tablet with obtained solid dispersion
   b) Mixing lactose monohydrate and microcrystalline cellulose,
   c) Mixing the solid dispersion at step (a) and the mixture at step (b) and done wet granulating,
   d) Sieving the wet granule,
   e) Drying the wet granule in a fluidized bed,
   f) Sieving the mixture,
   g) Adding Croscarmellose sodium and anhydrous colloidal silicon dioxide and then mixing,
   h) Adding magnesium stearate and then mixing,
   i) Compressing the mixture into the core tablet,
   j) Coating the core tablets with film coating agent.

### Example 1: Film coated tablet comprising empagliflozin

| **Ingredients** | **Amount (% by weight of the total core tablet)** |
|---|---|
| Empagliflozin or a pharmaceutically acceptable salt thereof or crystalline polymorph thereof | 0.5 - 25.0 |
| Lactose Monohydrate | 45.0 - 70.0 |
| Microcrystalline Cellulose (for example: PH 101) | 18.0 - 40.0 |
| Hydroxypropyl Cellulose (for example: LF) | 1.0 - 10.0 |
| Croscarmellose sodium | 1.0 - 10.0 |
| A surfactant (SLS or Tween) | 1.0 - 9.0 |
| Anhydrous colloidal silicon dioxide | 0.1 - 3.0 |
| Magnesium stearate | 0.1 - 3.0 |
| A solvent (IPA or water) | q.s. |
| **TOTAL CORE TABLET** | **100** |

### Example 2: Film coated tablet comprising empagliflozin

| **Ingredients** | **Amount (% by weight of the total core tablet)** | **Amount (% by weight of the total core tablet)** |
|---|---|---|
| Empagliflozin | 4.0 | 12.5 |
| Lactose Monohydrate | 64.0 | 54.5 |
| Microcrystalline Cellulose (for example: PH 101) | 25.0 | 25.0 |
| Hydroxypropyl Cellulose (for example: LF) | 3.0 | 3.0 |
| Croscarmellose sodium | 2.0 | 2.0 |
| A surfactant (SLS or polysorbate) | 1.0 | 2.0 |
| Anhydrous colloidal silicon dioxide | 0.5 | 0.5 |
| Magnesium stearate | 0.5 | 0.5 |
| A solvent (IPA or water) | q.s. | q.s. |
| **TOTAL CORE TABLET** | **100** | **100** |

A process for example 1 or 2;
Obtained solid dispersion
   a) Mixing surfactant (SLS or polysorbate), HPC, empagliflozin and a solvent (pure water or isopropyl alcohol) and obtained solid dispersion,
Preparing a tablet with obtained solid dispersion
   b) Mixing lactose monohydrate and microcrystalline cellulose,
   c) Mixing the solid dispersion at step (a) and the mixture at step (b) and done wet granulating,
   d) Sieving the wet granule,
   e) Drying the wet granule in a fluidized bed,
   f) Sieving the mixture,
   g) Adding Croscarmellose sodium and anhydrous colloidal silicon dioxide and then mixing,
   h) Adding magnesium stearate and then mixing,
   i) Compressing the mixture into the core tablet,
   j) Coating the core tablets with film coating agent.

## Claims

1. A tablet formulation comprising a solid dispersion of empagliflozin or a pharmaceutically acceptable salt thereof or crystalline polymorph thereof wherein the solid dispersion further comprising at least one binder, at least one surfactant and solvent.

2. The tablet according to claim 1, wherein binders are selected from the group comprising corn starch, hydroxypropyl cellulose, carboxymethylcellulose sodium, starch mucilage, acacia mucilage, dextrose, ethyl cellulose, glyceryl behenate, hydroxyethyl cellulose, hydroxyethyl methyl cellulose, hydroxypropyl starch, hypromellose, magnesium aluminum silicate, maltodextrin, maltose, methylcellulose, poloxamer, polydextrose, polyethylene oxide, polymethacrylates or mixtures thereof.

3. The tablet according to claim 2, wherein the binder is hydroxypropyl cellulose.

4. The tablet according to claim 1, wherein surfactants are selected from the group comprising polysorbates, sodium lauryl sulfate, docusate sodium, glyceryl esters, glyceryl monoleate, poloxamer, polyethylene alkyl ethers, polyglyceryl esters, polyoxyetylene esters, polyoxyetylene stearates, sodium stearate, calcium oleate or mixtures thereof.

5. The tablet according to claim 4, wherein the surfactant is sodium lauryl sulfate or polysorbate.

6. The tablet according to claim 1, wherein solvents are selected from the group comprising pure water, isopropyl alcohol, propylene glycol, polyethylene glycol, glycerin, ethanol, or mixtures thereof.

7. The tablet according to claim 6, wherein the solvent is pure water or isopropyl alcohol or mixtures thereof.

8. The tablet according to claim 1, wherein the tablet composition further comprises at least one pharmaceutically acceptable excipient is selected from the group comprising disintegrants, fillers, lubricants, glidant or mixtures thereof.

9. The tablet according to claim 8, wherein disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

10. The tablet according to claim 9, wherein the disintegrant is croscarmellose sodium.

11. The tablet according to claim 8, wherein fillers are selected from the group comprising lactose, lactose monohydrate, spray-dried lactose monohydrate, anhydrous lactose, microcrystalline cellulose, pregelatinized starch, mannitol, corn starch, maltodextrin, fructose, maltose, sucrose, dicalcium phosphate, ammonium alginate, calcium carbonate, calcium phosphate, calcium phosphate dehydrate, neutral pellets, cellulose, cellulose acetate, magnesium oxide, maltodextrin, medium chain triglycerides, polydextrose, polymethacrylates, sodium alginate, starch, sugar sphericals or mixtures thereof.

12. The tablet according to claim 11, wherein the fillers are microcrystalline cellulose and lactose monohydrate.

13. The tablet according to claim 8, wherein lubricants are selected from the group comprising magnesium stearate, calcium stearate, zinc stearate, talc, boric acid, hydrogenated vegetable oil, sodium chlorate, magnesium lauryl sulfate, polyethylene glycol, stearic acid, fumaric acid or mixtures thereof.

14. The tablet according to claim 8, wherein glidants are selected from the group comprising anhydrous colloidal silicon dioxide, calcium stearate, sodium chlorate, magnesium lauryl sulfate, sodium oleate, sodium acetate, sodium benzoate, polyethylene glycol, sodium lauryl sulfate, aluminum silicate, colloidal silica, calcium silicate or mixtures thereof.

15. The tablet according to claim 8, wherein comprising;
a) Empagliflozin or a pharmaceutically acceptable salt thereof or crystalline polymorph thereof
b) Microcrystalline Cellulose
c) Lactose monohydrate
d) HPC
e) Croscarmellose sodium
f) A surfactant
g) Anhydrous colloidal silicon dioxide
h) Magnesium stearate
